# EUROPÄISCHE PATENTANMELDUNG

(11) **EP 0 714 675 A2**
(43) Veröffentlichungstag der Anmeldung: **05.06.1996**
(21) Anmeldenummer: 95117119.8
(22) Anmeldetag: 31.10.1995
(51) Int. Cl.: A61M 39/22

(54) **Ventilvorrichtung**

(30) Priorität: 29.11.1994 DE 4442352
(71) Anmelder: B. BRAUN MELSUNGEN AG, D-34212 Melsungen (DE)
(72) Erfinder: Schmidt, Klaus-Joachim, D-34292 Ahnatal (DE); Fuchs, Jürgen, D-34308 Bad Emstal (DE); Wiegel, Heinz, D-36211 Alheim (DE)
(74) Vertreter: Selting, Günther, Dipl.-Ing.

(57) **Zusammenfassung**

Bei einer Ventilvorrichtung in einem Anschlußstück ist vorgesehen, daß der Ventilkörper als Ventilklappe (20) mit geschlossener Fläche ausgebildet ist, die über eine elastische radiale Zunge (21) in dem Anschlußstück (10) befestigt ist und in Schließstellung gegen eine ringförmige Stützfläche (26) anliegt, und daß der Schiebeteil die Ventilklappe (20) durch Druck gegen ihre geschlossene Fläche in Öffnungsrichtung um die radiale Zunge (21) verschwenkt.

## Beschreibung

Die Erfindung bezieht sich auf eine Ventilvorrichtung in einem Anschlußstück mit einem am vorderen und hinteren Ende offenen Durchflußkanal, in dem ein Ventilkörper angeordnet ist, der mittels eines axial verstellbaren Schiebeteiles in Öffnungsstellung bringbar ist.

Anschlußstücke mit Ventilvorrichtung sind im allgemeinen in eine Leitung eingebaut, deren Kanal in einer Richtung durchlässig und in entgegengesetzter Richtung abgesperrt sein soll. Der Begriff "Leitung" umfaßt dabei steife und flexible Kanülen sowie flexible Katheter, die im Körper eines Patienten verlegt sind, am einen Ende und Schlauchleitungen von Infusions- und Transfusionsvorrichtungen, Druckmeßleitungen sowie Spritzen am anderen Ende des Anschlußstückes, wobei die Ventilvorrichtung eine ungehinderte Zuführung oder Entnahme von Flüssigkeit ermöglichen und einen automatischen Verschluß dann bewirken soll, wenn der gewollte Flüssigkeitsstrom abbricht, damit kein Körpersekret das Umfeld des Anschlußstückes kontaminiert bzw. Luft in den Körper des Patienten eindringt. Beides ist für den Patienten gefährlich, weil es bakterielle Infektionen und die Gefahr von Embolien heraufbeschwört und ersteres kann durch Übertragung von Krankheitskeimen auch dem Anwender schaden.

Derartige Ventilvorrichtungen haben z. B. bei einer Arterienpunktion die Aufgabe, das gegenüber der Atmosphäre mit einem Überdruck von 100 - 200 mm Hg im Katheter befindliche Blut zurückzuhalten, wenn nach erfolgreicher Punktion der Arterie die scharf geschliffene Stahlkanüle aus dem Katheter herausgezogen wird. Weiterhin muß das Ventil sich öffnen und das Durchströmen von Blut bzw. eines fließenden Mediums ermöglichen, sobald der Luer-Konus einer Verbindungsleitung mit dem Anschlußstück konnektiert wird zur Entnahme von Blutproben oder zur Messung des Blutdruckes über die Flüssigkeitssäule.

Aus DE 28 17 102 C2 ist ein Anschlußstück mit einer Ventilvorrichtung bekannt, deren Ventilkörper aus einer Scheibe aus elastomerem Material mit einem zentralen Schlitz besteht. In dem Durchflußkanal ist ein relativ zu der Scheibe verstellbarer Körper gelagert, der vom Anschlußkonus eines Infusionsschlauches vorgeschoben wird und in vorgeschobener Stellung den Schlitz der Scheibe mindestens teilweise durchdringt, um ihn aufzuspreizen und offenzuhalten. Bei Freigabe des Schiebeteiles von dem Anschlußkonus soll die elastisch verformbare Scheibe den Schiebeteil zurückdrücken, wobei sich der Schlitz dichtend schließen soll. Die Verschlußgeschwindigkeit und Dichtigkeit der Ventilvorrichtung hängen dabei von der Rückstellfähigkeit des Scheibenmaterials und der Unversehrtheit der Schlitzklappen ab. Materialermüdung verringert daher die Effektivität. Diese ergibt sich bei mehrmaliger Öffnung der Ventilvorrichtung während eines Langzeiteinsatzes des Anschlußstückes. Außerdem macht sie sich bemerkbar bei Verwendung der Anordnung z. B. in einem Punktionsbesteck mit inliegender Stahlkanüle. In diesem Falle deformiert die Stahlkanüle die geschlossenen Schlitzlippen über einen längeren Zeitraum und es bildet sich durch plastische Verformung ein Loch, das sich nach Herausziehen der Stahlkanüle nicht schließt, so daß durch das Loch in dem an sich geschlossenen Schlitz Körpersekret nach außen dringt. Im übrigen ist die bekannte Ventilvorrichtung bei höheren Innendrücken, z. B. arteriellem Druck (Überdruck 200 mm WS), nicht zuverlässig, weil die Gefahr besteht, daß der Innendruck das geschlitzte Zentrum der Scheibe nach außen wölbt, so daß die Schlitzlippen nicht mehr auf ihrer ganzen Dicke fest aneinanderliegen, sondern nach außen divergieren. Dadurch wird die Dichtfläche so verringert, daß der arterielle Druck Blut nach außen pressen kann und ein Boden für Bakterienwachstum gebildet wird, das sich in den Patienten hinein fortsetzt und bakterielle Infektionen einschleust.

Eine aus EP-A-0 261 317 bekannte Ventilvorrichtung arbeitet ebenfalls mit einer Aufstoßmechanik und ihre Absperrleistung hängt von der Rückstellfähigkeit des als geschlossene Elastomerscheibe ausgebildeten Ventilkörpers ab. Die Elastomerscheibe liegt mit ihrer Innenfläche auf einer im Durchflußkanal angeordneten Lagerungsspitze und gegen ihre Außenfläche wirkt ein Schiebeteil, der zwei Gabelschenkel aufweist, die gegen den Randbereich der Elastomerscheibe drücken, um ihn von einem ringförmigen Ventilsitz wegzudrücken, damit der Durchflußkanal geöffnet wird. Zwar ist diese bekannte Ventilvorrichtung in Rückströmrichtung auch bei hohen Innendrücken dicht, jedoch gibt sie keinen zentralen Kanal frei, durch den ein langgestreckter Gegenstand, wie eine Stahlkanüle, ein Führungsdraht oder ein Katheter, hindurchgeführt werden könnte, und sie ist daher für diesen breiten medizinischen Produktbereich ungeeignet.

Der Erfindung liegt die Aufgabe zugrunde, eine Ventilvorrichtung in einem Anschlußstück so auszubilden, daß das Anschlußstück universell einsetzbar ist und sich durch zuverlässige Dichtigkeit sowohl bei höheren Drücken in Gegenströmrichtung als auch nach längerer Lagerzeit mit inliegendem langgestrecktem Teil auszeichnet.

Diese Aufgabe wird erfindungsgemäß dadurch gelöst, daß der Ventilkörper als Ventilklappe mit geschlossener Fläche ausgebildet ist, die über eine elastische radiale Zunge in dem Anschlußstück befestigt ist und in Schließstellung gegen eine ringförmige Stützfläche anliegt, und daß der Schiebeteil die Ventilklappe durch Druck gegen ihre geschlossene Fläche in Öffnungsrichtung um die radiale Zunge verschwenkt.

Diese Ventilvorrichtung arbeitet als Klappenventil mit Aufstoßmechanismus, so daß eine Öffnung nicht nur zur Zuführung von Flüssigkeit, sondern auch zur Entnahme möglich ist. Die Rückstellung der Ventilklappe in Schließposition wird durch den Körperinnendruck bewirkt, der die Ventilklappe fest gegen die ringförmige Stützfläche anpreßt. Die Durchbrechungslosigkeit der Ventilklappe gibt keine Möglichkeit für das Entstehen von Sickerdurchlässen unter höheren Innendrücken. Der Kanalverschluß ist zuverlässig wirksam und wird umso dichter, je höher der Innendruck ist. Das Aufhängen der Ventilklappe an der radialen elastischen Zunge befreit sie von jeglichen Trägheitskräften und sie schließt praktisch verzögerungslos in dem Moment, in dem der Innendruck auftritt, d. h. in dem rückströmende Flüssigkeit gegen die Ventilklappe trifft. Die Verschlußwirkung der Ventilklappe ist unabhängig von ihrer Eigenelastizität und dem Rückstellvermögen ihres Materials, weshalb sie beliebig oft öffenbar ist, ohne daß sich die Schließwirksamkeit verändert. Das Anschlußstück bleibt außen sauber und Patient und Anwender werden nicht durch Körperfluid infiziert. Da die Ventilklappe nur über die schmale elastische radiale Zunge in dem Anschlußstück befestigt ist, hängt sie bei Anwendung der Vorrichtung in einem Gefäßkatheter lose neben einem durch den Durchflußkanal hindurchgesteckten langgestreckten Teil (Stahlkanüle oder Mandrin) und wird von diesem überhaupt nicht verformt. Deshalb ist sie auch dann zuverlässig voll wirksam, wenn die Ventilvorrichtung in ein Anschlußstück für ein Katheterbesteck mit eingesetzter Stahlkanüle oder inliegendem Führungsdraht eingebaut ist, wobei der Durchmesser des langgestreckten Gegenstandes keine Rolle spielt und die Ventilvorrichtung somit auch für Stahlkanülen mit großem Außendurchmesser geeignet ist.

Der Schiebeteil kann ein Außenkonus eines Kupplungselementes oder einer Spritze sein. In diesem Falle ergibt sich eine sehr kleine Bauweise des Anschlußstückes und die Herstellungskosten sind gering, weil zusätzliche Einbauten entfallen.

In vorteilhafter Ausgestaltung der Erfindung ist der Schiebeteil als rohrförmiger Körper gestaltet, der im Durchflußkanal verschiebbar angeordnet und in diesem gegen Herausfallen gesichert ist. Der rohrförmige Körper wird mit Hilfe eines Spritzenkonus oder eines Außenkonus eines Leitungs-Kupplungsstückes vorgeschoben, um die Ventilklappe von der ringförmigen Stützfläche abzuheben und den Durchflußkanal von außen nach innen freizugeben. Da die an der elastischen radialen Zunge hängende Ventilklappe dem Druck des rohrförmigen Körpers praktisch keinen Widerstand entgegensetzt, braucht dieser keine besonderen Stabilitätseigenschaften zu haben und er kann ein leichtes dünnwandiges Teil sein. Der Kraftaufwand beim Verschieben des Schiebeteiles ist außerordentlich gering.

Die Ausgestaltungen gemäß den Ansprüchen 10 und 11 bilden Abdichtungen, die bei Verwendung eines eingesetzten langgestreckten Teiles wirksam sind, um die Zeit während des Zurückziehens des langgestreckten Teiles bis zum vollständigen Verschluß der Ventilklappe zu überbrücken. Die Dichtlippe bzw. die Dichtscheibe bildet eine Abdichtung um eine Stahlkanüle oder einen Mandrin, damit beim Herausziehen des langgestreckten Teiles Blutaustritt vermieden wird, solange sich seine Spitze noch im Bereich der Ventilklappe befindet.

Die ringförmige Stützfläche kann Teil des Anschlußstückes sein. Auch ist es möglich, die Stützfläche an einer zentral gelochten Dichtmembran auszubilden, die zwischen der Ventilklappe und dem Schiebeteil angeordnet und randseitig in dem Anschlußstück befestigt ist. Die Dichtmembran sorgt durch eine ebene großflächige Anlagebasis für die Ventilklappe einerseits für Steigerung der Abdichtwirkung an der Ventilklappe und andererseits hält sie die Ventilklappe von direkter Berührung durch den Schiebeteil frei, so daß ihre Dichtfläche auch nach häufigem Öffnungsvorgang frei von Kratz- oder Druckspuren bleibt. Außerdem beschleunigt sie die Rückführung der Ventilklappe in Schließstellung dadurch, daß sie den rohrförmigen Körper durch ihre Membranspannkraft elastisch zurückdrückt, so daß die vom Körperinnendruck beaufschlagte Ventilklappe sofort gegen eine in ihrer Schließebene liegende Stützfläche zur Anlage kommt und verkantungsfrei dicht ist.

Die Dichtmembran kann eine Scheibe aus Elastomermaterial sein. Bevorzugt ist die Scheibe auf beiden Seiten ebenflächig. Alternativ kann sie einen dickwandigen äußeren Flanschrand aufweisen, welcher in einer inneren Umfangsrille des Anschlußstückes aufgenommen ist. Mindestens auf der der Ventilklappe zugewandten Seite der Dichtmembran ist eine die zentrale Lochung umgebende Ringwulst ausgebildet, die als Stützfläche für die Ventilklappe dient.

Der rohrförmige Körper kann mit der Dichtmembran einstückig geformt sein. Dies ist herstellungs- und einbautechnisch günstig, weil mit der Montage der Dichtmembran gleichzeitig der rohrförmige Körper zentriert und passend genau in dem Anschlußstück untergebracht ist. Außerdem entfallen Dichtungsprobleme an der Führung des rohrförmigen Körpers.

Ventilklappe und elastische radiale Zunge können aus unterschiedlichen Materialien gefertigt sein. Die Ventilklappe kann aus steifem Material bestehen und mit einer elastischen ringförmigen Stützfläche zusammenwirken. Wichtig ist, daß die radiale Zunge elastisch ist, so daß sie der Bewegung der Ventilklappe unter dem Einfluß des Schiebeteiles bzw. des rückströmenden Fluids keinen Widerstand entgegensetzt. In vorteilhafter Ausgestaltung der Erfindung ist vorgesehen, daß die radiale Zunge die Ventilklappe mit einem Außenring verbindet, daß Zunge, Ventilklappe und Außenring einstückig aus elastischem Flachmaterial gebildet sind und daß der Außenring zwischen Ringflächen im Anschlußstück eingespannt ist, so daß er bei Anordnung zwischen zwei zusammengesteckten Gehäuseteilen diese nach außen abdichtet.

Weitere zweckmäßige Ausgestaltungen der Erfindung sind in Unteransprüchen gekennzeichnet.

Ausführungsbeispiele der Erfindung werden anhand der Zeichnung nachfolgend erläutert.

Es zeigt:

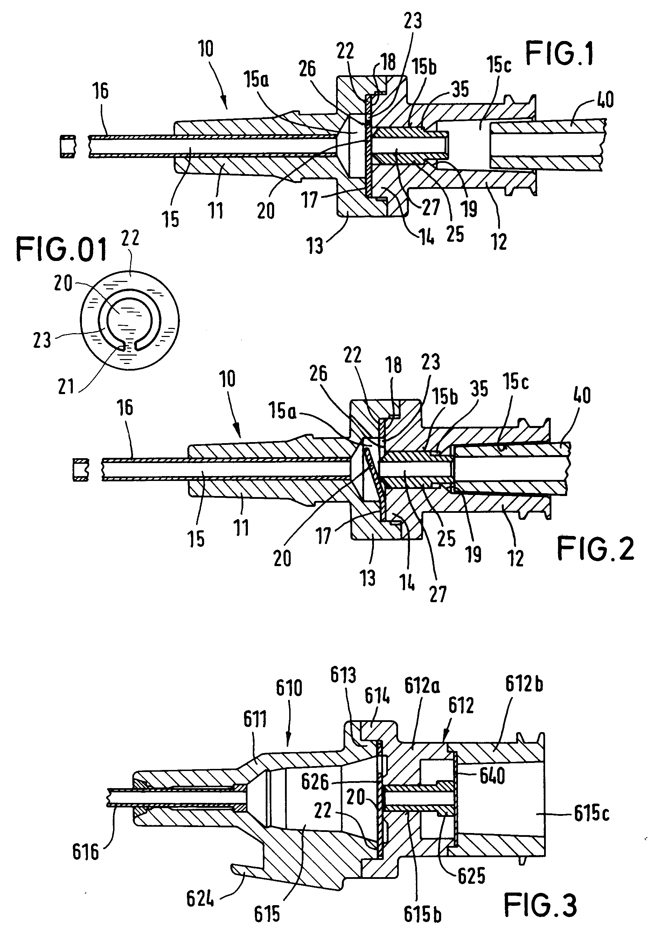

- Fig. 01: eine Draufsicht auf die Ventilklappenanordnung, die für sämtliche Ausführungsbeispiele verwendet wird,
- Fig. 1: einen Längsschnitt durch ein Anschlußstück mit einer ersten Ausführungsform einer Ventilvorrichtung im Schließzustand,
- Fig. 2: die Ventilvorrichtung im Öffnungszustand,
- Fig. 3: einen Längsschnitt durch ein Anschlußstück mit einer zweiten Ausführungsform einer Ventilvorrichtung im Schließzustand,
- Fig. 4: einen Längsschnitt durch ein Anschlußstück mit einer dritten Ausführungsform einer Ventilvorrichtung im Schließzustand,
- Fign. 5, 5A, 5B, 5C: Längsschnitte durch ein Anschlußstück mit einer vierten Ausführungsform einer Ventilvorrichtung in vier verschiedenen Zuständen,
- Fig. 6: einen Längsschnitt durch ein Anschlußstück mit einer fünften Ausführungsform einer Ventilvorrichtung im Schließzustand,
- Fig. 7: einen Längsschnitt durch ein Anschlußstück mit einer sechsten Ausführungsform einer Ventilvorrichtung im Schließzustand,
- Fig. 8: die Ventilvorrichtung nach Fig. 7 im Öffnungszustand und
- Fig. 9: einen Längsschnitt durch ein Anschlußstück mit einer siebenten Ausführungsform einer Ventilvorrichtung.

Das Beispiel der Figuren 1 bis 3 zeigt die einfachste Version einer Ventilvorrichtung mit eingebautem Schiebeteil in einem Anschlußstück. Das Anschlußstück 10 besteht aus einem Gehäuseoberteil 11 und einem Gehäuseunterteil 12 aus Kunststoff, die an flanschartigen Steckteilen 13,14 durch Ultraschallschweißung miteinander verbunden sind. Gehäuseoberteil 11 und Gehäuseunterteil 12 sind von koaxialen Durchgängen durchsetzt, die einen an beiden Enden offenen Durchflußkanal 15 des Anschlußstückes 10 bilden. Am äußeren Ende des Gehäuseunterteiles 12 befinden sich Vorsprünge einer Luer-Lock-Verriegelung. In einem kreiszylindrischen Abschnitt des Durchflußkanals 15 im Gehäuseoberteil 11 ist eine starre oder flexible Kanüle befestigt, die eine Stahlkanüle mit scharfer Spitze oder ein Katheterschlauch 16 sein kann. Der Durchflußkanal 15 ist im Mittelbereich 15a erweitert. Von dort aus setzt er sich nach hinten in einen engeren kreiszylindrischen Führungsabschnitt 15b fort, an den sich ein nach hinten offener Innenkonus 15c anschließt.

In dem Anschlußstück 10 ist eine Ventilvorrichtung untergebracht. Sie besteht bei allen Ausführungsbeispielen aus einer in Fig. 3 gezeigten kreisförmigen Ventilklappe 20, die über eine radiale Zunge 21 mit einem Außenring 22 verbunden ist, wobei sich zwischen dem Außenumfang der Ventilklappe 20 und dem Innenumfang des Außenringes 22 ein Ringraum 23 befindet, der an den beiden geraden Flanken der radialen Zunge 21 endet. Die Gesamtanordnung 20 bis 23 ist einstückig aus elastischem Flachmaterial gebildet. Die Ventilklappe 20 ist in Bezug auf den Außenring 22 quer zu seiner Ebene beweglich, wobei die geringe Breite der radialen Zunge 21 der Bewegung praktisch keinen Widerstand entgegensetzt und im wesentlichen als Halte- und Zentrierelement dient. Der Außenring 22 ist zwischen zwei Ringflächen 17 und 18 des Gehäuseoberteiles 11 und des Gehäuseunterteiles 12 eingeklemmt, so daß er sich nicht verschieben kann. Die radiale Breite der Ringfläche 17 ist dem Außenring 22 angepaßt. Konzentrisch zu der Ventilklappe 20 ist in dem Führungsabschnitt 15b des Durchflußkanales 15 ein rohrförmiger Körper 25 axial verschiebbar vorgesehen, der am hinteren Ende eine Stufe 35 aufweist, die mit einer Anschlagrippe 19 am hinteren Ende des Führungsabschnittes 15b zusammengreift, damit der rohrförmige Körper 25 nicht aus dem Durchflußkanal 15 herausfällt. In dieser Position stößt das vordere, verjüngte Ende des rohrförmigen Körpers 25 an die Rückseite der Ventilklappe 20 an, die dicht an eine ringförmige Stützfläche 26 am Steckteil 14 des Gehäuseunterteiles 12 anliegt. Dies hat den Vorteil, daß der rohrförmige Körper nicht zusätzlich zum Gehäuseunterteil 12 in dem Führungsabschnitt 15b abgedichtet werden muß und ein sehr leichter Schiebesitz ausgeführt werden kann. Der rohrförmige Körper 25 besteht aus Kunststoff und ist von einer Bohrung 27 durchsetzt, deren Durchmesser etwa dem Durchmesser des Lumens des Katheterschlauches 16 entspricht. Das hintere Ende des rohrförmigen Körpers 25 ist stumpf und ragt ein kurzes Stück in den Innenkonus 15c hinein.

Nach Punktion eines Blutgefäßes, in das der Katheterschlauch 16 eingeführt werden soll, mittels einer die Ventilklappe 20 seitlich wegdrückenden, durch den Durchflußkanal 15 ragenden Stahlkanüle wird die Stahlkanüle entfernt und es trifft rückströmendes Blut gegen die Ventilklappe 20. Der Blutrückstrom drückt die Ventilklappe 20 gegen die Stützfläche 26 am Steckteil 14 und verschließt den Durchflußkanal 15. Um die Ventilklappe 20 für Fluideinlaß von außen oder Fluidentnahme von innen zu öffnen, wird in den Innenkonus 15c ein passender hohler Außenkonus 40 eingesteckt, der Teil einer Spritze oder eines Kupplungselementes sein kann, das eine Verbindung zwischen dem Katheterschlauch 16 und einer Schlauchleitung herstellt, die zu einem Flüssigkeitsübertragungsgerät oder einem Meßgerät führt (Fig. 2). Der Außenkonus 40 wird so weit in den Innenkonus 15c vorgeschoben, bis er gegen das hintere Ende des rohrförmigen Körpers 25 trifft und diesen ein Stück nach vorne verschiebt. Dabei wird die Ventilklappe 20 um die radiale Zunge 21 verschwenkt und von der Stützfläche 26 abgehoben. Unter dem Druck der von hinten anströmenden Flüssigkeit wird die an der radialen Zunge 21 hängende Ventilklappe 20 in dem erweiterten Mittelbereich 15a weiter zur Seite gedrückt und der volle Querschnitt des Durchflußkanals 15 steht dem Flüssigkeitsstrom zur Verfügung. Sobald der Flüssigkeitsstrom in den Patientenkörper aufhört und der Außenkonus 40 von dem Anschlußstück 10 abgekoppelt wird, tritt rückströmendes Blut in den Katheterschlauch 16 ein, drückt gegen die Ventilklappe 20 und preßt sie gegen die Stützfläche 26 an dem Gehäuseunterteil 12, wobei der rohrförmige Körper 25 seine hintere Anschlagposition einnimmt. Je höher der Innendruck ist, umso dichter ist der Verschluß. Es tritt praktisch kein Blut durch die Bohrung 27 in den Innenkonus 15c ein.

Die Ventilvorrichtung gemäß der zweiten Ausführungsform nach Fig. 3 ist in ein Anschlußstück 610 mit einem Durchflußkanal 615 eingebaut, das tangential anschliessende seitliche Flügel 624 zur Befestigung auf der Haut aufweist und als Ansatz einer Arterienkanüle 616 geeignet ist. Das Anschlußstück 610 besteht aus einem Gehäuseoberteil 611 und einem Gehäuseunterteil 612, wobei das Gehäuseunterteil 612 aus einem Frontstück 612a und einem Endstück 612b zusammengesetzt ist, die zwischen Steckteilen eine zum Durchlaß einer Stahlkanüle oder eines Mandrins zentral gelochte Dichtscheibe 640 straff eingespannt halten. Zur Verbindung des Gehäuseoberteiles 611 mit dem Gehäuseunterteil 612 dienen Steckteile 613 und 614, die durch Ultraschallschweißung miteinander verbunden sein können. Zwischen Ringflächen 617 und 618 des Gehäuseoberteiles 611 und des Gehäuseunterteiles 612 ist der Außenring 22 der Ventilklappe 20 fest eingespannt.

Koaxial zu der geschlossenen Ventilklappe 20 erstreckt sich im Durchflußkanal 615 ein rohrförmiger Körper 625, der in einem zylindrischen Führungsabschnitt 615b verschiebbar ist und - wie in Verbindung mit Fign. 1 und 2 erläutert - der Öffnung mit der Ventilklappe 20 bzw. ihrer Freigabe zur Schließung unter Blutrückstrom dient. Im Schließzustand liegt die Ventilklappe 20 an einer Stützfläche 626 an, die an einer inneren Stirnfläche des Frontstückes 612a ausgebildet ist. Das hintere Ende des rohrförmigen Körpers 625 stützt sich an der Dichtscheibe 640 ab, die bei hindurchgestecktem langgestrecktem Teil den Durchflußkanal 615 nach hinten abdichtet. Ein in die konische Öffnung 615c des Endstückes 612b hinter der Dichtscheibe 640 eingesteckter Außenkonus ähnlich dem Außenkonus 40 gemäß Fign. 1 und 2 drückt gegen die elastische Dichtscheibe 640 und durch diese hindurch gegen den rohrförmigen Körper 625, wodurch die Ventilklappe 20 entsprechend der in Fig. 2 veranschaulichten Konstellation geöffnet wird. In diesem Zustand ist sie von der ringförmigen Stützfläche 626 abgehoben. Blutrückstrom schließt die Ventilklappe 20 wieder und stellt den rohrförmigen Körper 625 zurück.

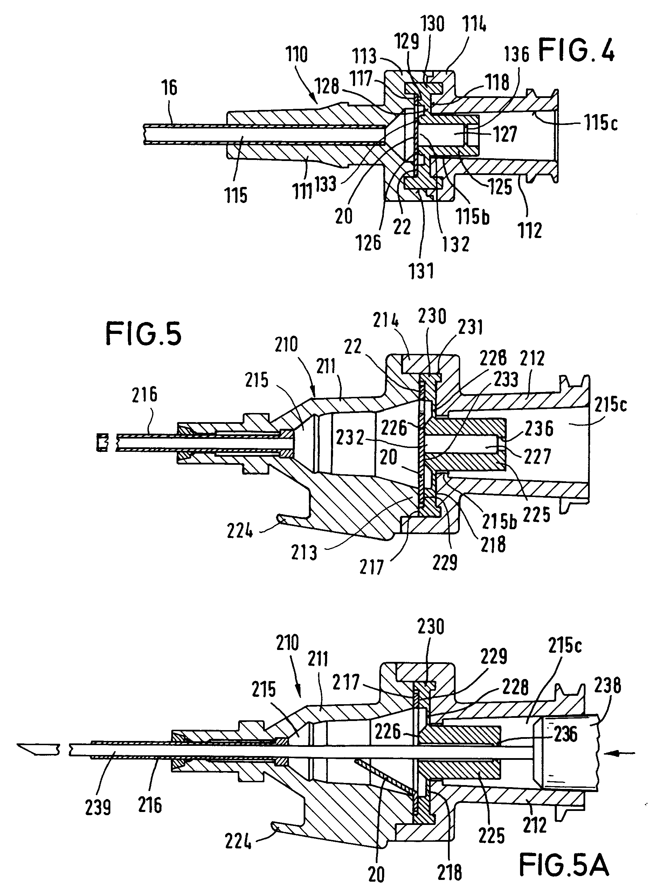

Die Ventilvorrichtung des in Fig. 4 gezeigten Beispieles ist derjenigen des Beispieles der Figur 1 ähnlich. Auch hierbei ist in ein Anschlußstück 110 mit einem Katheterschlauch 16 und einem Durchflußkanal 115 die Ventilklappe 20 eingebaut, die über die radiale Zunge 21 mit dem Außenring 22 verbunden ist (Fig. 01). Ein rohrförmiger Körper 125 mit einer koaxialen Bohrung 127 ist bei diesem Beispiel aus elastischem Material gefertigt und einstückig mit einer Dichtmembran 128 ausgebildet, die mit einem zu der Bohrung 127 konzentrischen Loch 132 versehen ist, das von einer Ringwulst 133 umgeben ist, die die Stützfläche 126 aufweist. Am hinteren Ende des rohrförmigen Körpers 125 ist eine einwärts gerichtete, radiale Dichtlippe 136 ausgebildet, die als umfangsmäßige Abdichtung für einen Mandrin oder eine Stahlkanüle dient, die durch den Durchflußkanal 115 hindurchgesteckt werden können. Die Dichtmembran 128 weist einen dickwandigen äußeren Flanschrand 130 auf, der sich in Achsrichtung nach beiden Seiten erstreckt und in einer angepaßten inneren Umfangsrille 131 in Steckteilen 113, 114 in der Trennzone zwischen dem Gehäuseoberteil 111 und dem Gehäuseunterteil 112 des Anschlußstückes 110 aufgenommen ist.

Die Einheit des rohrförmigen Körpers 125 und der Dichtmembran 128 wird gemeinsam mit der Ventilklappenanordnung 20 - 23 eingebaut bevor die beiden Gehäuseteile 111 und 112 miteinander verbunden und verschweißt werden. Diese Montage ist sehr einfach und garantiert präzisen Sitz der Teile. Der Außenring 22 der Ventilklappenanordnung ist zwischen der Ringfläche 117 des Gehäuseoberteiles 111 und einer an den Flanschrand 130 radial einwärts anschließenden Stirnfläche 129 eines axial vorspringenden Radialabschnittes der Dichtmembran 128 eingespannt. Die Rückseite der Dichtmembran 128 ist ebenflächig und liegt gegen eine kreisförmige Ringfläche 118 des Gehäuseunterteiles 112 an. Die Dichtmembran 128 dichtet den Übergang an der Einspannung der Ventilklappenanordnung ab und hält die Führung 115b für den rohrförmigen Körper 125 frei von Körpersekret. Außerdem hat die Dichtmembran 128 die Aufgabe, den rohrförmigen Körper 125 durch ihre Membranspannkraft beschleunigt nach hinten zu verstellen, wenn er von dem Außenkonus 40 freikommt. Auf diese Weise wird die Rückstellgeschwindigkeit der Ventilklappe 20 unter dem Druck rückströmenden Blutes gesteigert.

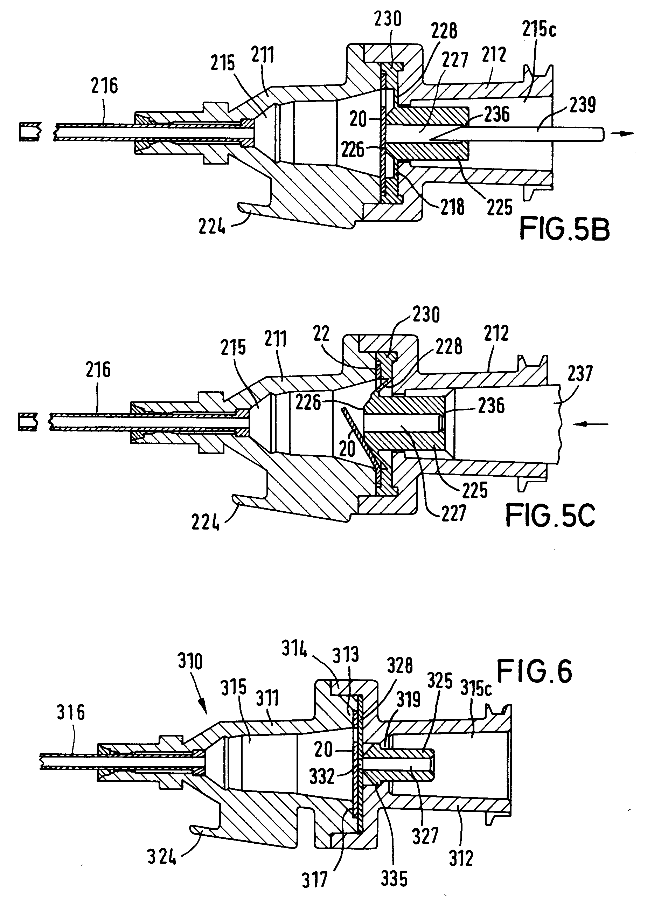

Das Beispiel der Figuren 5, 5A, 5B, 5C zeigt die Ventilvorrichtung bei einem Anschlußstück 210 mit tangential angeschlossenen seitlichen Flügeln 224 zur Befestigung auf der Haut, das als Ansatz zur Befestigung einer Kunststoffkanüle bzw. eines Katheters 216 geeignet ist. Das Anschlußstück 210 setzt sich aus einem Gehäuseoberteil 211 und einem Gehäuseunterteil 212 zusammen, die an Steckteilen 213,214 zusammengesteckt und miteinander vorzugsweise durch Ultraschallschweißung verbunden sind. Das Anschlußstück 210 ist von einem durchgehenden Kanal 215 durchsetzt, der im Bereich des Gehäuseoberteiles 211 nach hinten abschnittweise erweitert ist und im Bereich des Gehäuseunterteiles 212 als Innenkonus 215c zum Anschluß eines Außenkonus 237 einer Spritze oder eines Kupplungselementes einer Schlauchleitung bzw. eines Ansatzes 238 einer Stahlkanüle 239 gestaltet ist. Am hinteren Ende des Innenkonus 215c befinden sich nach außen gerichtete Gewindeteile einer Luer-Lock-Verriegelung.

In der Verbindungszone zwischen dem oberen und dem unteren Gehäuseteil 211 und 212 ist die Ventilklappe 20 mit Außenring 22 (Fig. 01) angeordnet. Der Außenring 22 wird zwischen einer Ringfläche 217 des Gehäuseoberteils 211 und einer Stirnfläche 229 eines Flanschrandes 230 eingespannt, der Teil einer Dichtmembran 228 ist. Die Dichtmembran 228 ist einstückig mit einem kreiszylindrischen rohrförmigen Körper 225 aus Elastomermaterial hergestellt, so daß eine vollkommene axiale Abdichtung durch radiale Überspannung des Durchflußkanals 215 erreicht wird. Ihr äußerer dicker Flanschrand 230 ist in einer angepaßten inneren Umfangsrille 231 des Gehäuseoberteiles 212 aufgenommen. Der rohrförmige Körper 225 ist von einer Bohrung 227 durchsetzt, die koaxial zu einem Loch 232 in der Mitte der Dichtmembran 228 angeordnet ist. Das Loch 232 ist auf der der Ventilklappe 20 zugewandten Seite von einer Ringwulst 233 mit der Stützfläche 226 umgeben. Der rohrförmige Körper 225 ragt mit dem größten Teil seiner Länge in einen Innenkonus 215c des Gehäuseunterteils 212 hinein. Als Führung für den rohrförmigen Körper 225 dient ein axial kurzer zylindrischer Führungsabschnitt 215b. Die Innenkante des Führungsabschnittes 215b schließt sich an eine radial nach außen gerichtete Kreisringfläche 218 an. Diese dient als Anlagefläche für die Dichtmembran 228. Am hinteren Ende des rohrförmigen Körpers 225 befindet sich eine radial einwärts gerichtete Dichtlippe 236, die die Bohrung 227 verengt.

Der Außenring 22 der Ventilklappe 20 steht mit der dahinter angeordneten Dichtmembran 228 formschlüssig in Verbindung und bildet somit die äußere Dichtung des zweigeteilten Anschlußstückes 210. Der rohrförmige innere Körper 225 in der Dichtmembran 228 bildet im Bereich der Gehäusedichtung mit seiner Stirnfläche im Zusammenwirken mit der Ventilklappe 20 die flüssigkeitsdichte Abdichtung in Richtung des Innenkonus 215c.

Die ringförmige Dichtlippe 236 dichtet bei einer Gefäßpunktion die Stahlkanüle 239 ab, die das Anschlußstück 210 und den Katheter 216 durchsetzt und die Ventilklappe 20 in Öffnungsstellung seitlich wegdrückt (Fig. 5A). Beim Zurückziehen der Stahlkanüle 239 ist die Dichtlippe 236 wirksam, bis der komplette Kanülenschliff die Ventilklappe 20 passiert hat (Fig. 5B). Durch den Flüssigkeitsdruck des arteriellen Blutes wird die Ventilklappe 20 beim Herausziehen der Stahlkanüle 239 unmittelbar auf die Dichtfläche der Dichtmembran 238 gedrückt und verhindert somit ein Austreten des Blutes. Im Neuzustand eines solchen Katheterisierungsbesteckes hängt die Ventilklappe 20 lose neben der Stahlkanüle und bleibt von jeglichen Deformationen, die später Dichtigkeitsprobleme hervorrufen könnten, frei.

Sobald in den Innenkonus 215c ein Außenkonus 237 z. B. einer Druckleitung oder eines Mehrwegehahnes eingesteckt wird, drückt die Stirnfläche des Außenkonus 237 den rohrförmigen Körper 225 in Richtung der Katheterspitze bzw. Gefäßzuganges und hebt damit die Ventilklappe 20 von der Stützfläche 226 ab, so daß das flüssige Medium in Vorwärts-Richtung bzw. das Blut in Rückwärts-Richtung den Durchflußkanal 215 durchströmen kann (Fig. 5C).

Nach Herausziehen des Außenkonus 237 und in der Grundstellung im Neuzustand ergibt sich die in Fig. 5 gezeigte rückgestellte Position des rohrförmigen Körpers 225, in der sein die Stützfläche 226 für die Ventilklappe 20 bildendes vorderes Ende in die Ebene der Rückfläche der Ventilklappe 20 zurückgezogen ist und bei gegen die Ventilklappe 20 wirkendem Innendruck ein dichter Verschluß des Durchflußkanales 215 erreicht wird.

Gemäß Fig. 6 ist ein Anschlußstück 310 aus einem Gehäuseoberteil 311 und einem Gehäuseunterteil 312 zusammengesetzt. Eine radial einwärts gerichtete Anschlagrippe 319 im Gehäuseunterteil 312 definiert die zurückgezogene Position eines als Einzelteil ausgebildeten rohrförmigen Körpers 325 mit einer Bohrung 327, der in Grundposition mit einer nach außen gerichteten Stufe 335 an die Anschlagrippe 319 anliegt. Die Anschlagrippe 319 besitzt einen axial kurzen, kreiszylindrischen Führungsabschnitt für den kreiszylindrischen Schaftteil des rohrförmigen Körpers 325. Das vordere konische Ende des rohrförmigen Körpers 325 liegt in Grundstellung gegen eine als ebenflächige Scheibe ausgebildete Dichtmembran 328 an, die in der Mitte ein Loch 332 besitzt, dessen Durchmesser im wesentlichen dem Durchmesser der Bohrung 327 des rohrförmigen Körpers 325 entspricht. Der äußere Rand der Dichtmembran 328 ist eingefaßt zwischen Paßflächen des Steckteiles 314 des Gehäuseunterteils 312 und des Steckteiles 313 des Gehäuseoberteils 311. Gegen die Dichtmembran 328 liegt die Ventilklappe 20 mit ihrer Halterung 21,22 flächig an. Ihr Außenring 22 wird von der Ringfläche 317 des Steckteiles 313 gegen den Randbereich der Dichtmembran 328 gepreßt, der an der Stirnfläche des Gehäuseunterteiles 312 abgestützt ist.

Beim Einstecken eines Außenkonus 40 in den Innenkonus 315c verschiebt sich der rohrförmige Körper 325 nach vorne, das Zentrum der Dichtmembran 328 erhält eine Wölbung nach vorne und drückt die Ventilklappe 20 in den aufgeweiteten Teil des Durchflußkanals 315, so daß das Loch 332 freikommt und ein freier Fließstrom durch die Arterienkanüle 316 ermöglicht wird. Nach Zurückziehen des Außenkonus 40 drückt die Spannkraft der Dichtmembran 328 den rohrförmigen Körper 325 unverzüglich zurück, so daß die von rückströmendem Blut beaufschlagte Ventilklappe 20 gegen die absolut ebene Stützfläche an der Dichtmembran 328 trifft und sofort das Loch 332 verschließt. Die flächige Anlage der Ventilklappe 20 gegen die Dichtmembran 328 sorgt für hervorragende Abdichtung.

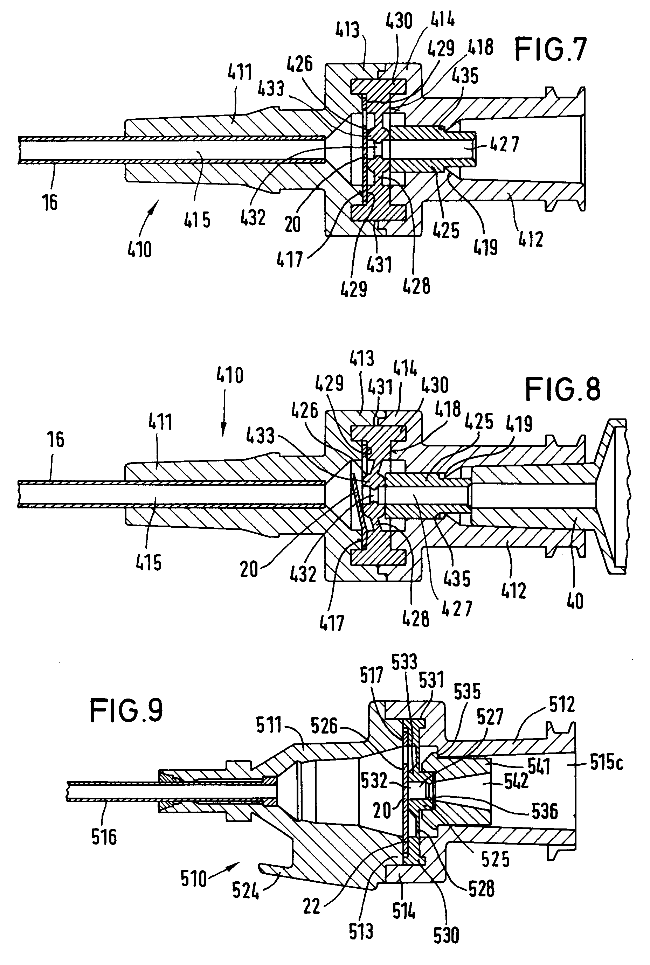

Das Beispiel der Figuren 7 und 8 verwendet ein dem Anschlußstück 110 der Fig. 4 ähnliches Anschlußstück 410, dessen Gehäuseoberteil 411 in einem Durchflußkanal 415 einen Katheterschlauch 16 aufweist. In der Verbindungszone zwischen dem Gehäuseoberteil 411 und dem Gehäuseunterteil 412 ist in Steckteilen 413 und 414 eine Umfangsrille 431 zur Aufnahme eines dickwandigen äußeren Flanschrandes 430 einer Dichtmembran 428 ausgebildet.

Die Dichtmembran 428 besteht aus Elastomermaterial. An den Flanschrand 430 schließt sich ein radial einwärts gerichteter Ringkörper an, dessen Außenfläche gegen eine Ringfläche 418 des Gehäuseunterteils 412 anliegt und dessen Innenfläche eine Stirnfläche 429 bildet, gegen die der Außenring 22 der Ventilklappenanordnung (Fig. 3) von der Ringfläche 417 des Gehäuseoberteils 411 angepreßt wird. Ein an den Ringkörper anschließender dünner Abschnitt der Dichtmembran 428 ist in der Mitte mit einem Loch 432 versehen, das zu der Bohrung 427 des rohrförmigen Körpers 425 konzentrisch und auf beiden Seiten umgeben ist von einer balligen Ringwulst 433. Die Ringwulst 433 bildet auf der Außenseite ein elastisches Widerlager für die Stirnfläche des rohrförmigen Körpers 425, der ein separates Teil aus Kunststoff darstellt, das am hinteren Ende eine Stufe 435 aufweist, die mit einer Anschlagrippe 419 des Gehäuseunterteiles 412 zusammengreift, wenn die Dichtmembran 428 und die Ventilklappe 20 die in Fig. 7 gezeigte Grundstellung (Schließposition) eingenommen haben. Der nach vorne gerichtete innere Teil der Ringwulst 433 dient als Stützfläche 426 für die Ventilklappe 20 und sorgt unter dem Anstrom rückfließenden Blutes für elastische Abdichtung. Die ballige Ausbildung der Ringwulst 433 im Bereich der radialen Zunge 21 der Ventilklappe 20 vermittelt eine Vergrößerung des Öffnungswinkels der Ventilklappe 20, wenn der rohrförmige Körper 425 von einem Außenkonus 40 vorgeschoben wurde und der Durchgang für Injektat oder zur Blutentnahme freigegeben ist.

Bei der siebenten Ausführungsform gemäß Fig. 9 ist ein Anschlußstück 510 ähnlich aufgebaut, wie das Anschlußstück 210 der Figuren 5 - 5C. An einem Gehäuseoberteil 511 sind tangential angeschlossene seitliche Flügel 524 zur Befestigung auf der Haut ausgebildet und mit dem vorderen Ende ist ein Katheterschlauch 516 fest verbunden. Ein Gehäuseunterteil 512 ist über Steckteile 513 und 514, die durch Ultraschallschweißung aneinander befestigt sein können, mit dem Gehäuseoberteil 511 verbunden. In der Trennfuge zwischen den beiden Teilen befindet sich eine Dichtmembran 528 aus elastischem Material, deren äußerer Randbereich verdickt ausgebildet ist und einen dicken Flanschrand 530 aufweist, der in eine angepaßte Umfangsrille 531 des Gehäuseunterteiles 512 eingesetzt ist. Mit dem dünnwandigen Zentrum der Dichtmembran 528 ist einstückig ein rohrförmiger Körper 525 ausgebildet, dessen zentrale Bohrung 527 in ein Loch 532 der Dichtmembran 528 übergeht. Das Loch 532 ist auf der Vorderseite der Dichtmembran 528 von einer Ringwulst 533 umgeben, die die Stützfläche 526 für die Ventilklappe 20 bildet. Der Außenring 22 der Ventilklappe 20 ist eingespannt zwischen einer Ringfläche 517 des Gehäuseoberteiles 511 und einer Fläche des Randbereiches der Dichtmembran 528.

Das hintere Ende des sehr kurz gehaltenen rohrförmigen Körpers 525 weist eine gegen die Mittelachse gerichtete geschlossene Dichtlippe 536 auf, die die gleiche Aufgabe wie die Dichtlippe 236 des Beispiels der Figuren 5 bis 5C hat. Ein separater Stößel 541 überfängt mit seinem vorderen kappenförmigen Ende den rohrförmigen Körper 525 und zentriert ihn auf der Längsachse des Anschlußstückes 510, so daß er trotz seines geringen Durchmessers und seiner Aufhängung in der Dichtmembran 528 kippsicher gehalten ist und beim Hindurchschieben eines langgestreckten Teiles nicht verkantet. Der Stößel 541 ist vorzugsweise aus festem Kunststoff hergestellt und in dem Innenkonus 515C des Gehäuseunterteiles 512 geführt. Eine nach außen gerichtete Stufe 535 am Kopfende des Stößels 541 wirkt mit einer Kante des Gehäuseunterteiles 512 als Anschlag zusammen, der ein Herausfallen des Stößels 541 verhindert. Der Stößel 541 ist von einem zum anderen Ende von einer Bohrung 542 durchsetzt, die sich gegen den rohrförmigen Körper 525 verjüngt. Diese Ausbildung der Ventilvorrichtung in einem Anschlußstück verkürzt die Bauweise des Anschlußstückes bei stabiler Ausführung und hervorragender Abdichtung durch das Zusammenwirken der Ventilklappe 20, der Dichtmembran 528 und der Dichtlippe 536.

## Patentansprüche

1. Ventilvorrichtung in einem Anschlußstück mit einem am vorderen und hinteren Ende offenen Durchflußkanal, in dem ein Ventilkörper angeordnet ist, der mittels eines axial verstellbaren Schiebeteiles in Öffnungsstellung bringbar ist,
**dadurch gekennzeichnet,**
daß der Ventilkörper als ventilklappe (20) mit geschlossener Fläche ausgebildet ist, die über eine elastische radiale Zunge (21) in dem Anschlußstück (10) befestigt ist und in Schließstellung gegen eine ringförmige Stützfläche (26) anliegt,
und daß der Schiebeteil die Ventilklappe (20) durch Druck gegen ihre geschlossene Fläche in Öffnungsrichtung um die radiale Zunge (21) verschwenkt.

2. Ventilvorrichtung nach Anspruch 1, dadurch gekennzeichnet, daß der Schiebeteil ein Außenkonus eines Kupplungselementes oder einer Spritze ist.

3. Ventilvorrichtung nach Anspruch 1, dadurch gekennzeichnet, daß der Schiebeteil als rohrförmiger Körper (25) gestaltet ist, der im Durchflußkanal (15) verschiebbar angeordnet und in diesem gegen Herausfallen gesichert ist.

4. Ventilvorrichtung nach einem der Ansprüche 1 bis 3, dadurch gekennzeichnet, daß die Stützfläche (26;626) an einer inneren Stirnfläche des Anschlußstückes (10;610) im Bereich des Durchflußkanals ausgebildet ist.

5. Ventilvorrichtung nach einem der Ansprüche 1 bis 3, dadurch gekennzeichnet, daß die Stützfläche (126;226;426; 526) an einer zentral gelochten Dichtmembran (128;228; 328;428;528) ausgebildet ist, die zwischen der Ventilklappe (20) und dem Schiebeteil (125;225;325;425;525) angeordnet und randseitig in dem Anschlußstück (110;210; 310;410;510) befestigt ist.

6. Ventilvorrichtung nach Anspruch 5, dadurch gekennzeichnet, daß die Dichtmembran (328) eine Scheibe aus Elastomermaterial ist.

7. Ventilvorrichtung nach Anspruch 5 oder 6, dadurch gekennzeichnet, daß die Dichtmembran (128;228;428;528) einen dickwandigen äußeren Flanschrand (130;230;430;530) aufweist, welcher in einer inneren Umfangsrille (131;231; 431;531) des Anschlußstückes (110;210;410;510) aufgenommen ist.

8. Ventilvorrichtung nach einem der Ansprüche 5 bis 7, dadurch gekennzeichnet, daß die Dichtmembran (128;228;428; 528) mindestens auf der der Ventilklappe (20) zugewandten Seite eine die zentrale Lochung (132;232;432;532) umgebende Ringwulst (133;233;433;533) aufweist.

9. Ventilvorrichtung nach einem der Ansprüche 3 bis 7, dadurch gekennzeichnet, daß der rohrförmige Körper (125; 225;525) mit der Dichtmembran (128;228;528) einstückig geformt ist.

10. Ventilvorrichtung nach einem der Ansprüche 3 bis 9, dadurch gekennzeichnet, daß am hinteren Ende des rohrförmigen Körpers (125;225;525) eine innerhalb der Bohrung (127;227;527) einwärts gerichtete radiale Dichtlippe (136;236;536) ausgebildet ist.

11. Ventilvorrichtung nach einem der Ansprüche 3 bis 10, dadurch gekennzeichnet, daß der Durchgangskanal (615) hinter dem rohrförmigen Körper (625) von einer Dichtscheibe (640) überspannt ist.

12. Ventilvorrichtung nach einem der Ansprüche 1 bis 10, dadurch gekennzeichnet, daß die radiale Zunge (21) die Ventilklappe (20) mit einem Außenring (22) verbindet, daß Zunge (21), Ventilklappe (20) und Außenring (22) einstückig aus elastischem Flachmaterial gebildet sind, und daß der Außenring (22) zwischen Ringflächen (17,18) im Anschlußstück (10) eingespannt ist.

13. Verwendung der Ventilvorrichtung gemäß den Ansprüchen 1 bis 12 bei einem Gefäßkatheter, bei dem in dem Durchflußkanal (215) des Anschlußstückes (210) eine Punktionskanüle (239) oder ein Mandrin steckt, die bzw. der die Ventilklappe (20) in Öffnungsstellung hält, ohne den Schiebeteil zu betätigen.
